(19) **Europäisches Patentamt / European Patent Office / Office européen des brevets**

(11) **EP 2 699 159 B1**

(12) ## EUROPEAN PATENT SPECIFICATION

(45) Date of publication and mention of the grant of the patent:
**06.04.2016 Bulletin 2016/14**

(21) Application number: **11863775.0**

(22) Date of filing: **09.08.2011**

(51) Int Cl.:
*A61B 5/06* (2006.01)     *A61B 5/0275* (2006.01)
*G01N 21/64* (2006.01)     *A61B 1/04* (2006.01)
*A61B 1/307* (2006.01)

(86) International application number:
**PCT/KR2011/005801**

(87) International publication number:
**WO 2012/144696 (26.10.2012 Gazette 2012/43)**

(54) **PROSTATE CANCER DIAGNOSIS DEVICE USING FRACTAL DIMENSION VALUE**

VORRICHTUNG ZUR PROSTATAKREBSDIAGNOSE MIT FRAKTALEM DIMENSIONSWERT

DISPOSITIF DE DIAGNOSTIC DU CANCER DE LA PROSTATE UTILISANT UNE VALEUR DE DIMENSION FRACTALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **20.04.2011   KR 20110036609
20.04.2011   KR 20110036608
02.06.2011   KR 20110053506
02.06.2011   KR 20110053504**

(43) Date of publication of application:
**26.02.2014 Bulletin 2014/09**

(73) Proprietors:
• **IM Co., Ltd.**
  **Gyeonggi-do 445-170 (KR)**
• **Fraunhofer Gesellschaft zur Förderung der angewandten Forschung E.V.**
  **80686 München (DE)**

(72) Inventors:
• **LEE, Sang Dae**
  **Suwon-si**
  **Gyeonggi-do 443-373 (KR)**
• **KIM, Kyung Sik**
  **Anyang-si**
  **Gyeonggi-do 431-085 (KR)**
• **BAIK, Jun Heum**
  **Wonju-si**
  **Gangwon-do 220-130 (KR)**
• **KIM, Eunjung**
  **Yongin-si**
  **Gyeonggi-do 446-731 (KR)**

• **HAN, Tae-Young**
  **01109 Dresden (DE)**
• **GERICH, Carola**
  **01109 Dresden (DE)**
• **ZEH, Christoph**
  **01109 Dresden (DE)**
• **HAERTLING, Thomas**
  **01109 Dresden (DE)**
• **OPITZ, Joerg**
  **01109 Dresden (DE)**
• **SCHREIBER, Juergen**
  **01109 Dresden (DE)**

(74) Representative: **Winter, Brandl, Fürniss, Hübner, Röss, Kaiser, Polte - Partnerschaft mbB Patent- und Rechtsanwaltskanzlei Bavariaring 10 80336 München (DE)**

(56) References cited:
**WO-A1-2010/130254       WO-A2-2009/059072
US-A1- 2004 044 287       US-A1- 2009 009 759
US-A1- 2009 270 702       US-B2- 7 016 718**

• **GERICH C E ET AL: "Detection of cancer cells in prostate tissue with time-resolved fluorescence spectroscopy", OPTICAL INTERACTIONS WITH TISSUE AND CELLS XXII, SPIE, 1000 20TH ST. BELLINGHAM WA 98225-6705 USA, vol. 7897, no. 1, 10 February 2011 (2011-02-10), pages 1-12, XP060007307, DOI: 10.1117/12.876094 [retrieved on 2011-02-22]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- FELEKYAN S ET AL: "Full correlation from picoseconds to seconds by time-resolved and time-correlated single photon detection", REVIEW OF SCIENTIFIC INSTRUMENTS, AIP, MELVILLE, NY, US, vol. 76, no. 8, 21 July 2005 (2005-07-21), pages 83104-083104, XP012079605, ISSN: 0034-6748, DOI: 10.1063/1.1946088

- WEISS M ET AL: "Anomalous protein diffusion in living cells as seen by fluorescence correlation spectroscopy", BIOPHYSICAL JOURNAL, CELL PRESS, US, vol. 84, no. 6, 1 June 2003 (2003-06-01), pages 4043-4052, XP002550854, ISSN: 0006-3495, DOI: 10.1016/S0006-3495(03)75130-3

**Description**

**Technical Field**

The present invention relates to a prostate cancer diagnosis device using a fractal dimension value ($D_F$) value of temporal processes in prostate cells detected by autofluorescence, and more particularly, the present invention relates to a prostate cancer in-vivo diagnosis device using a fractal dimension value.

**Background Art**

In general, a conventional medical device for diagnosing prostate cancer is a light source supply device using a gas laser, and particularly, a nitrogen ($N_2$) gas laser. The nitrogen gas laser is operated with a range of 375 nm ultraviolet rays and is pumped by electric discharge, and has a merit of being inexpensive.

However, the nitrogen gas laser has an unstable output due to an irregular pulse amplitude, and accordingly measured data values are unstable so that sensitivity with respect to a diagnosis result is low. In addition, a conventional medical device for diagnosing prostate cancer is large in size because of using a gas laser and spectroscopy.

Further, since the medical device for diagnosing prostate cancer uses a biomarker, a relatively long period of time is required to acquire a diagnosis result, and it has a problem of being exposed to radical rays in a diagnosis using a radioactive isotope.

In addition, when diagnosing prostate cancer by a pathologist, there is a risk of false positives, and when this occurs, there is a significantly longer time between diagnoses and treatment.

The above information disclosed in this Background section is only for enhancement of understanding of the background of the invention and therefore it may contain information that does not form the prior art that is already known in this country to a person of ordinary skill in the art.

The present invention has been made in an effort to provide a prostate cancer diagnosis device using a fractal dimension value ($D_F$) of temporal processes in prostate cells detected by auto-fluorescence that promptly provides an accurate and objective diagnosis result on a prostate cancer.

Further, the present invention provides a prostate cancer diagnosis device using a $D_F$-value of temporal processes in prostate cells that can diagnose a prostate cancer not by using a biomarker but by using a semiconductor laser, and a method thereof.

The Article "Detection of Cancer Cells in Prostate Tissue with Time-Resolved Fluorescence Spectroscopy" by C.E. Gerich et al ("Optical Interactions with Tissue and Cells XXII", SPIE, 1000 20th St. Bellingham WA 98225-6705 USA; vol. 7897, no. 1, 10 February 2011) relates to cancer diagnosis wherein time-resolved fluorescence spectra were recorded for 4 different measurement points for each biopsy, and an algorithm was developed to determine a relevant parameter of the time dependent fluorescence data (fractal dimension DF). The results of the finding and the DF -value were correlated for each point and then analysed with statistical methods.

WO 2010/13254 A1 relates to a method and a device for detecting tumorous living cell tissue in collected samples of living cell tissue. In the method, a radiation source is used to emit electromagnetic radiation in a locally defined manner onto cell tissue and, after the radiation source has been switched off, the decay behaviour of the inherent fluorescence intensity excited in the cell tissue by the electromagnetic radiation is detected on the cell tissue by a detector, with temporal and spectral resolution and with a known scanning rate or known scanning rates for at least one wavelength. With the measured intensity values that are obtained, a difference autocorrelation function C(t) of the intensity decay behaviour is determined, from which the fractal dimension $D_F$ for the respective irradiated cell tissue is calculated, and the value of the fractal dimension $D_F$ is used for a classification in respect of a tumorous invasion of the respective irradiated cell tissue.

In WO 2009/059072 A2, methods and computer program products for analyzing tissue are provided. The tissue is exposed to light at the appropriate wavelengths for inducing fluorescence. Images of the fluorescing tissue are taken at two or more depths within the tissue. The PSD function is determined for each image at a different depth within the tissue. A characteristic of each PSD function determined is compared, and it is determined whether or not the tissue exhibits a pre-cancerous characteristic.

**Solution to Problem**

As defined in claim 1, the present invention provides a prostate cancer diagnosis device using a $D_F$-value of temporal processes in prostate cells. The prostate cancer diagnosis device includes: a semiconductor laser emitting a laser beam with a wavelength of = 375 nm; an endoscope transmitting the laser beam to a biopsy tissue in a human body and receiving auto-fluorescence generated from the biopsy tissue; an optical unit transmitting the laser beam to the endoscope and receiving an autofluorescence generated from the biopsy tissue from the endoscope; a detection unit detecting the auto-fluorescence received by the optical unit and measuring the single photon signal corresponding to a single pulse to obtain intensity of the time-dependent auto-fluorescence (hereinafter referred to as an auto-fluorescence measured value); and a diagnosis unit calculating $D_F$-values of temporal processes in prostate cells by the measured time dependent auto-fluorescence decay behaviour using an algorithm which yield a fractal dimension value.

The diagnosis unit further includes a function to determine a Gleason score for a biopsy core derived by all determined valued of the fractal dimension for the biopsy sample, usually 14 DF-values are available.

When the auto-fluorescence measured value is I(t), the fractal dimension algorithm includes: selecting a maximum I(t) that is higher than a predetermined reference intensity $I_{ref}$ as a target; approximation modeling the selected I(t) to a linear value using a modeling function F(t); calculating a correlation function that indicates a difference obtained by subtracting a measured value of an attenuation period of I(t) from the maximum value Fmax of F(t); modeling this correlation function with a fractal model and then discussing the correlation function in a double log-log coordinate system; there calculating a parameter related to the fractal dimension corresponding to the slope (hereinafter referred to as a first slope) of the correlation function in the log-log coordinate system, the first slope being determined by a start time and a variable time length; while first the variable time length is fixed, calculating a first fractal dimension value by changing the start time and determining the first start time corresponding to the minimal value of the calculated first fractal dimension value; and while using this start time, calculating a second fractal dimension value by changing the variable time length and determining the minimal value of the second derivative ot the calculated second fractal dimension value with respect to the variable time length.

Another, unclaimed exemplary embodiment provides a prostate cancer diagnosis method using DF-value of temporal processes in prostate cells. The prostate cancer diagnosis method includes: transmitting an diode laser beam with a wavelength of = 375 nm to a prostate sample and receiving auto-fluorescence generated from the sample of prostate tissue; detecting the auto-fluorescence received by an optical unit and measuring the single photon signal corresponding to a single pulse to obtain intensity of the time-dependent auto-fluorescence (hereinafter referred to as an autofluorescence measured value); and calculating the $D_F$-value of temporal processes in prostate cells by the measured time dependent auto-fluorescence decay behaviour using a algorithm which yield a fractal dimension value.

The prostate cancer diagnosis method according to the exemplary embodiment of the present invention may further include determining a Gleason score corresponding to the calculated fractal dimension value.

When the auto-fluorescence measured value is I(t), the calculating of the fractal dimension values includes: a first sub-step of selecting a maximum I(t) that is higher than a predetermined reference intensity $I_{ref}$ as a target; a second sub-step of approximation modeling the selected I(t) to a linear value using a modeling function F(t); a third sub-step of calculating a correlation function that indicates a difference obtained by subtracting a measured value of an attenuation period of I(t) from the maximum value $F_{max}$ of F(t); a fourth sub-step of modeling this correlation function with a fractal model and then discussing the correlation function in a double log-log coordinate system; a fifth sub-step of calculating a parameter related to the fractal dimension corresponding to the slope (hereinafter referred to as a first slope) of the correlation function in the log-log coordinate system, the first slope being determined by a start time and a variable time length; a sixth sub-step of calculating a first fractal dimension value by changing the start time and determining a first start time corresponding to the minimal value of the calculated first fractal dimension value while first the variable time length is fixed; and a seventh sub-step of calculating a second fractal dimension value by changing the variable time length and determining the minimal value of the second derivative of the calculated second fractal dimension value with respect to the variable time length.

**Advantageous Effects of Invention**

According to the exemplary embodiment of the present invention, time for diagnosing a prostate cancer can be minimized so that time for diagnosis to treatment can be remarkably shortened.

In addition, according to the exemplary embodiment of the present invention, an objective diagnosis result on tissue biopsy using a semiconductor laser method that is unharmful to a human body can be provided.

Further, according to the exemplary embodiment of the present invention, a patient does not have rejection on the prostate cancer diagnosis because it can be performed by minimal invasively and painlessly, the prostate cancer diagnosis is convenient to pathologists, and tension and irritation in an operating room can be solved.

Brief Description of Drawings

FIG. 1 is a block diagram of a prostate cancer diagnosis device, according to an exemplary embodiment of the present invention, and of a method of diagnosis.
FIG. 2 is a block diagram of an optical configuration of the prostate cancer diagnosis device according to the exemplary embodiment of the present invention.

FIG. 3 is a detailed diagram of the optical configuration of the prostate cancer diagnosis device according to the exemplary embodiment of the present invention.

FIG. 4 is a flowchart of a prostate cancer diagnosis method using a $D_F$-value according to the exemplary embodiment of the present invention.

FIG. 5 is a flowchart of a process for calculating the $D_F$-value according to the exemplary embodiment of the present invention.

FIG. 6 is a graph showing a measured value and a modeling value according to the exemplary embodiment of the present invention.

FIG. 7 is a graph showing a correlation between measured values in an attenuation period with reference to Fmax according to the exemplary embodiment of the present invention.

FIG. 8 is a graph of the correlation replaced into a log dimension according to the exemplary embodiment of the present invention.

FIG. 9 is a graph for determining a start time through fractal dimension values while a variable time is fixed according to the exemplary embodiment of the present invention.

FIG. 10 is a graph for determining fractal dimension values through fractal dimension values and differentiation values while the start time is fixed according to the exemplary embodiment of the present invention.

FIG. 11 is a graph of a correlation between the fractal dimension values and Gleason scores according to the exemplary embodiment of the present invention.

## Mode for the Invention

In the following detailed description, only certain exemplary embodiments of the present invention have been shown and described, simply by way of illustration. The drawings and description are to be regarded as illustrative in nature and not restrictive. Like reference numerals designate like elements throughout the specification.

Hereinafter, a prostate cancer diagnosis device using a fractal dimension value and a method thereof according to an exemplary embodiment of the present invention will be described in detail.

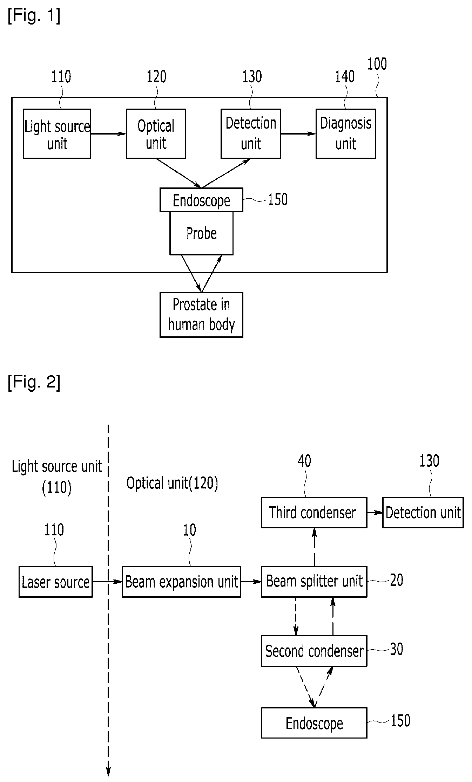

FIG. 1 is a block diagram of an in-vivo diagnosis device according to an exemplary embodiment of the present invention. As shown in FIG. 1, a prostate cancer in-vivo diagnosis device 100 using a TCSPC method according to the exemplary embodiment of the present invention includes a light source unit 110, an optical unit 120, a detection unit 130, a diagnosis unit 140, and an endoscope 150.

The light source unit 110 generates a diode laser beam and emits the same. The diode laser beam emitted from the light source unit 110 has a wavelength of 375nm or 750nm.

Here, the light source unit 110 uses one wavelength when emitting the diode laser beam is having a 375nm laser beam, and uses two wavelengths when emitting the diode laser beam having a 750nm laser beam. That is, when the light source unit 110 uses a diode laser beam having a 375nm wavelength, the light source unit 110 emits the laser beam once, and when the light source unit 110 uses a diode laser beam having a 750nm wavelength, the light source unit 110 emits the laser beam two times.

Since the diode laser beam having a 750nm wavelength is longer than the diode laser beam of 375nm wavelength in wavelength, the diode laser beam having a 750nm wavelength has a merit in measurement of the inside of prostate cancer compared to the diode laser beam having a 375nm wavelenfth that measures the surface portion of the prostate cancer. However, the diode laser beam of a 750nm wavelength has a lower wavelength energy compared to the laser beam of a 375nm wavelength.

Since the energy of a 750nm wavelength is lower than that of a 375nm wavelength, no or completely different fluorescence light is emitted from the sample A so that inaccurate measurement occurs.

In order to solve such a drawback, the light source unit 110 irradiates the laser beam of 750nm, and the laser beam is iteratively irradiated with a time interval of picoseconds to femtoseconds. That is, the light source unit 110 continuously irradiates two laser beams of 750nm (i.e., a pair of laser beams) with a time interval of picoseconds to femtoseconds.

Alternatively, the light source unit 110 may continuously irradiate the laser beams with a time interval of longer than femtoseconds.

When the pair of laser beams of 750nm are continuously irradiated with a time interval of picoseconds or femtoseconds, energy of each of the laser beams of a 750nm wavelength overlap each other such that the irradiation has an effect of irradiating the laser beam of a 375nm wavelength time interval.

The optical unit 120 transmits the laser beam of the light source unit 110 to the endoscope 150 for condensing of the beam to a prostate in human body, and receives an auto-fluorescence from the prostate and transmits the received one to the detection unit 130.

Here, protein of the prostate sample A includes a unique fluorescent material, called nicotinamide adenine dinucleotide (NADH), and NADH has an auto-fluorescence characteristic when an laser beam is irradiated thereto. NADH is a reduced form of nicotinamide adenine dinucleotide (NAD). NAD is widely used in the corresponding process in cell respiration and a tricarboxylic acid cycle (TCA), and a reduction potential stored in NADH is converted to adenosine 5-triphophate (ATP) while passing through an electron transport system or used for anabolism.

In prostate cells NADH is normally bound to cell-proteins, the structure of which is fractal and different for healthy and cancerous cells. Ecxiting the electrons in NADH by the 375 nm-laser beam, the excitation interacts with the vibrational and configurational states of the bound proteins. In such a way the differences in the prostate cell protein structure and dynamical behaviour may become visible in the time dependence of the fluorescence of NADH fluorophores.

The detection unit 130 detects photons of the auto-fluorescence using a photon multiplier tube (PMT) or a high-speed photo director, and detects the amount of auto-fluorescence emission (i.e., intensity) by the technique of time correlated single photon counting (TCSPC). A measured intensity of the prostate detected by the detection unit 130 will now be referred to as I(t).

The diagnosis unit 140 calculates a fractal dimension value $D_F$ from the intensity measured by the detection unit 130 using a fractal algorithm. A Gleason score for the biopsy core will be calculated taking into account all determined fractal dimension values $D_F$ for this core.

That is, the DF-values has corresponding Gleason grades (first grade to fifth grade), and the Gleason score is calculated from the sum of two Gleason grades. In this case, the diagnosis unit 140 stores a matching table of correlations between fractal dimension values and Gleason grades to determine a Gleason grade corresponding to a fractal dimension value.

In general, the Gleason grade is a technical term indicating a grade of tumor, and the grade indicates a differentiation degree of cancer cells. The Gleason grade is divided into the first grade to the fifth grade, and the fifth grade has highest possibility of cancer.

Typically, a pathologist uses the Gleason score as an indicator to determine prostate cancer probability.

When a Gleason score is determined, the diagnosis unit 140 determines a diagnosis result corresponding to the Gleason score and outputs the diagnosis result. Then, a user can determine the diagnosis result.

Here, reliability of the correlation between the fractal dimension values $D_F$ and the Gleason score has been proven through many experiments by many pathologists. A characteristic experiment is performed using the prostate sample as a target.

The diagnosis unit 140 can also display a fractal dimension value $D_F$ without using a matching table (i.e., without using a Gleason score) or can provide a diagnosis result indicating a grade of prostate cancer corresponding to a $D_F$-value. In this case, the diagnosis result indicating the grade of prostate cancer is stored corresponding to a fractal dimension value (or, a fractal dimension value range).

The endoscope 150 irradiates the laser beam provided through the optical unit 120 to prostate tissues, and receives auto-fluorescence emitted from the prostate tissues and provides the received auto-fluorescence to the detection unit 130. The endoscope 150 may be a filter scope or a needle scope, but the present invention is not limited thereto.

Hereinafter, an optical configuration of the prostate cancer in-vivo diagnosis device 100 according to the exemplary embodiment of the present invention will be described with reference to FIG. 2. FIG. 2 is a block diagram of the optical configuration of the prostate cancer diagnosis device according to the exemplary embodiment of the present invention.

As shown in FIG. 2, the optical configuration according to the exemplary embodiment of the present invention includes a light source unit 110 and an optical unit 120.

The light source unit 110 is formed of a semiconductor laser source, and may irradiate a laser beam of a 375nm wavelength or may irradiate two laser beams of a 750nm wavelength with a time interval of picoseconds or femtoseconds.

The optical unit 120 includes a beam expansion unit 10 expanding the laser beam and making the expended laser beam travel straight, a beam splitter 40 divaricating a path of the beam in a direction of the endoscope 150 and transmitting auto-fluorescence received from a prostate to a third condenser 40, a second condenser 30, and a third condenser 40. The second and third condensers 30 and 40 condense incident light.

Here, the second condenser 30 is disposed at the front of an emission cable (or, optical fiber) of the endoscope 150 to condense incident light to the emission cable. The third condenser 40 is disposed at the front of a detector cable (or, optical fiber) of endoscope 150. The emission cable and the detector cable transmit light.

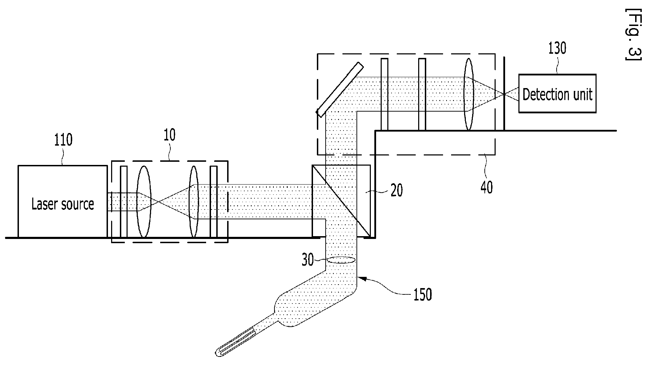

FIG. 3 is a detailed diagram of the optical configuration of the prostate cancer diagnosis device according to the exemplary embodiment of the present invention. As shown in FIG. 3, the beam expansion unit 10 of the optical unit 120 is sequentially formed of a radiation cleaning filter, a condensing lens, a beam expander, a lens transmitting light with constant intensity, and a halfwave plate. The beam expansion unit 10 cleans the laser beam of the semiconductor laser source 110, expands the cleansed beam to a constant size, and then causes the expanded beam to enter into the beam splitter unit 20.

Here, the beam expander functions to increase the size of the incident beam. The half-wave plate rotates a polarization direction of light 180 degrees, and it functions to increase reflectivity by changing the beam from a p-wave to an s-wave in a light transmission system. That is, the half-wave plate increases light efficiency.

The beam splitter unit 20 is formed of a beam splitter cube, and changes a path of light incident from the beam expansion unit 10 to the endoscope 150. In further detail, the beam splitter cube transmits light incident from the beam expansion unit 10 to the second condenser 30 disposed at the front of the endoscope 150, and transmits an auto-fluorescence incident from the endoscope 150 to the third condenser 40.

The second condenser 30 is disposed at the front end of the endoscope 150, and is formed of at least one cylindrical lens. The at least one cylindrical lens forming the second condenser 30 changes the shape of the beam in only one direction. That is, the cylindrical lens is used to adjust the shape of the beam provided to the emission cable of the endoscope 150.

The third condenser 40 receives an auto-fluorescence of the prostate tissue, detected through the detector cable of the endoscope 150 through the second condenser 30 and the beam splitter20, and provides the received auto-fluorescence to the detection unit 130.

In further detail, the third condenser 40 includes a reflection mirror, a notch filter, a polfilter, a confocal lens, and a field stop. The reflection mirror transmits the light incident from the beam splitter cube 20 to the notch filter, the notch filter corrects an impedance increase of a resonance frequency in the auto fluorescence, and the confocal lens brings the focus on the detection unit 130, that is, the confocal lens determines the size of the beam when a focal point is placed in the detection unit 130.

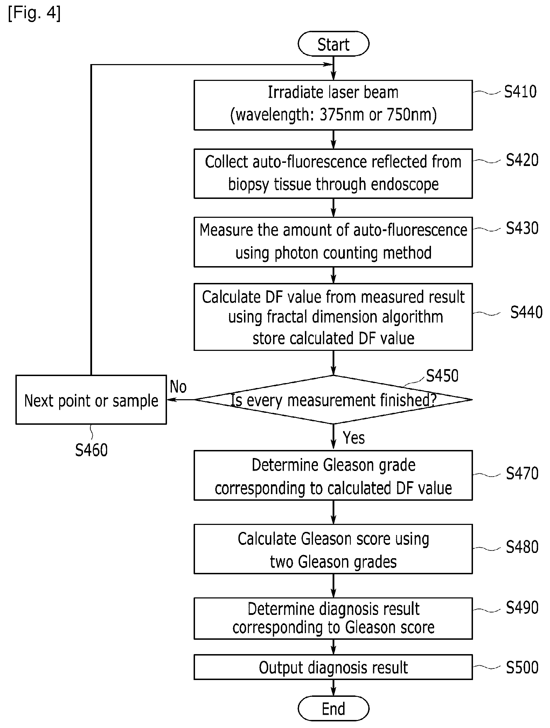

Hereafter, a prostate cancer diagnosis method using a $D_F$-value according to the exemplary embodiment of the present invention will be described with reference to FIG. 4. FIG. 4 is a flowchart of a prostate cancer diagnosis method using a $D_F$-value according to the exemplary embodiment of the present invention.

Initially, a plurality of prostate samples A are placed at a measurement location on a measurement shelf, and then a $D_F$-value of a plurality of measurement points for each of the plurality of prostate samples A are determined. For example, in the prostate cancer diagnosis method using the $D_F$-value according to the exemplary embodiment of the present invention, a biopsy is performed in 12 directions with respect to a prostate tissue extracted from a human body to acquire a prostate sample for measurement and a $D_F$-value for each of 12 prostate samples A is determined with a regular interval of 14 points. The number of prostate samples A to be measured may be more or less than 12, and the number of measurement points may be more or less than 14.

As shown in FIG. 4, a diode laser having a wavelength of 375nm or 750nm is irradiated from the semiconductor laser source 110 (S410).

The irradiated diode laser beam is condensed to the prostate tissue (biopsy tissue) through the optical unit 120 and the endoscope 150, and accordingly, an auto-fluorescence is emitted from the prostate tissue by the diode laser beam and the auto-fluorescence is collected in the detection unit 130 through the endoscope 150 and the optical unit 120 (S420).

In this case, the detection unit 130 detects the auto-fluorescence using a high-speed photodetector or a photon multiplier tube (PMT), and measures intensity (the amount of emission) of a single photon corresponding to a single pulse using a TCSPC method.

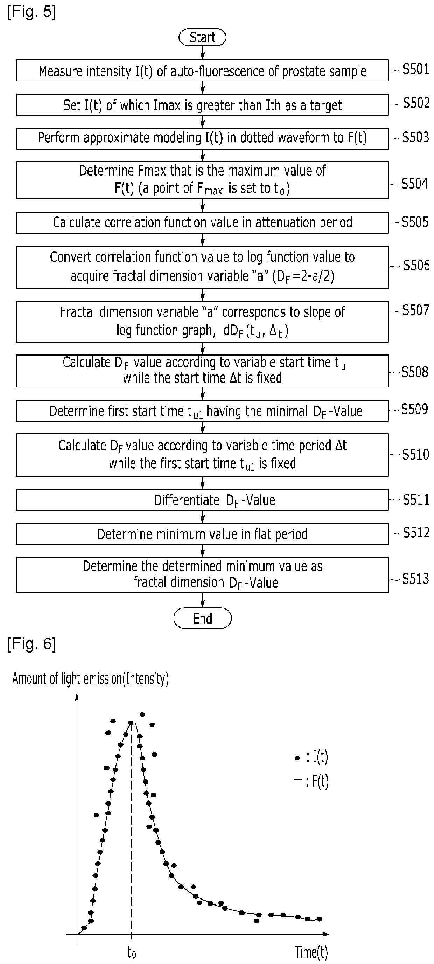

The intensity of the single photon is represented as a dot-shaped graph in the time axis as shown in FIG. 6, and the graph will be referred to as I(t) (S430). I(t) is a measured value of a time-dependent photon.

The diagnosis unit 140 calculates a fractal dimension value $D_F$ from the dot-shaped auto-fluorescence intensity graph I(f) using a fractal dimension algorithm and stores the calculated fractal dimension value $D_F$ (S440).

The diagnosis unit 140 determines whether a currently measured sample and a measurement point are the last measurement to determined termination of all measurements (S450).

If the measurement is not the last measurement, the diagnosis unit 140 controls a sample on the shelf for measurement or a measurement point to be the next target to be measured (S460), and then controls the steps S410 to S450 to be repeated. However, if the measurement is the last measurement, the diagnosis unit 140 determines a Gleason grade corresponding to a fractal dimension value of each prostate sample A and the measurement point using the matching table (S470).

In addition, the diagnosis unit 140 sums two Gleason grades according to conditions predetermined by an operator among the determined Gleason grades so as to calculate a Gleason score (S480).

In this case, the conditions are a Gleason score of a prostate sample A expected to be the highest Gleason grade (e.g., a voluntary Gleason score or the highest or lowest Gleason score) and a Gleason score of a prostate sample A expected to be the highest Gleason grade (e.g., a voluntary Gleason score or the highest or lowest Gleason score), but the conditions can be modified by the operator.

The diagnosis unit 140 determines a diagnosis result corresponding to the corresponding Gleason score using the calculated Gleason scores (S490), and outputs the determined diagnosis result on a screen or on paper for the operator (S500).

Hereinafter, operation of the diagnosis unit 140 that determines a fractal dimension value using the auto-fluorescence I(t) received from the prostate according to the exemplary embodiment of the present invention will be described with reference to FIG. 5.

FIG. 5 is a flowchart of a calculation process of the fractal dimension value according to the exemplary embodiment of the present invention. As shown in FIG. 5, a method for calculating the fractal dimension value $D_F$ using a fractal dimension algorithm is as follows.

The diode laser beam of the semiconductor laser source 11 is irradiated to each measurement point of a prostate core in a human body and the intensity I(t) of the auto-fluorescence generated from the prostate sample A is measured using a single photon counting method (S501).

Through such a measurement, the measure values intensity I(t) of the auto-fluorescence are represented as a dot-shaped curved line as shown in FIG. 6.

The diagnosis unit 140 selectively inputs the maximum intensity $I_{max}$ of higher than 100cps among the measured intensity I(t). Here, since a step S502 is performed, a process for selecting only the maximum intensity $I_{max}$ of higher than 100cps among the measured intensity I(t) can be omitted.

In addition, the diagnosis unit 140 compares the maximum intensity $I_{max}$ with a re-predetermined reference intensity $I_{ref}$, and sets a measured intensity I(t) of which the maximum intensity $I_{max}$ is higher than the reference intensity $I_{ref}$ as a target for testing (S502).

The diagnosis unit 140 performs approximation modeling with respect to measured value I(t) having a dotted waveform, set as a target for testing to represent the measured value in a curved line of Equation 1 (S503) (refer to FIG. 6).

Here, the step S503 uses the approximation modeling that is performed to acquire consistent results by eliminating an invalid measured value.

A function F(t) for initial intensity modeling is as given in Equation 1.

(Equation 1)

$$F(t) = \frac{A \boxminus \exp\left\{-\left(\left[\dfrac{(t-t_0)}{t_{ca}}\right]^2\right)^{\frac{a}{2}}\right\}}{\left(1+\exp\left[\dfrac{-(t-t_0)}{t_{cb}}\right]^2\right)^{\frac{b}{2}}}$$

The diagnosis unit 140 determines $F_{max}$ by repeating evaluation a predetermined number of times (e.g., three times) with respect to a waveform of the function F(t), and determines a time location $t_0$ of $F_{max}$ (S504).

Once $F_{max}$ and the time location $t_0$ of $F_{max}$ are determined, a correlation between the respective measured values (dot values) is determined based on $F_{max}$ using Equation 2.

(Equation 2)

$$C(t) = F_{max} \, I(t), \text{ with } t > t_o$$

Here, C(t) denotes a correlation function. In this case, the equation is performed in a time period where $t > t_0$ to determine a correlation in an attenuation falling curved line of F(t) (i.e., attenuation falling curved line of I(t)) (S505).

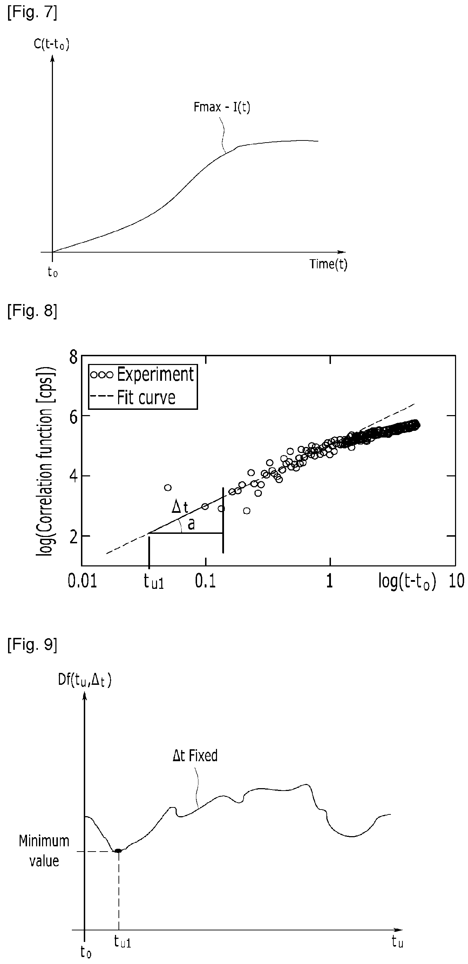

Since such a correlation function C(t) is based on a time location $t_0$ of $F_{max}$, the time location $t_0$ of $F_{max}$ is moved to the original location when being shifted to the left by $t_0$, and accordingly, $C(t-t_0)$ becomes as shown in FIG. 7.

FIG. 7 is a graph of correlations between measured values of one attenuation period with reference to $F_{max}$ according to the exemplary embodiment of the present invention. As shown in FIG. 7, the value of the correlation $C(t-t_0)$ is gradually increased in proportional to a distance from the time location $t_0$ and then converged to a constant value from a given time point.

Such a curved shape of the correlation function $C(t-t_0)$ (i.e., the shape of correlation) is influenced by a variable a of the function F(t) in Equation 1, and the variable a is a variable of the fractal dimension value $D_F$.

Thus, modeling of Equation 2 in association with the fractal dimension value $D_F$ can be given in Equation 3. A modeled fractal dimension value $D_F$ in Equation 3 is $D_F = 2-a/2$.

(Equation 3)

$$C(t-t_0) = C1 + C2(t - t_0)^a$$

Here, the fractal dimension value is calculated to be $D_F = 2 - a/2$, and accordingly the variable a in Equation 3 should be calculated.

For this, the diagnosis unit 140 replaces the correlation function of Equation 3 with a log function as shown in Equation 4 (S506). In a first step the constant C1 was chosen C1 = 0

(Equation 4)

$$\log(C(t-t_o) = C_o + a \log(t-t_o) \text{ , with } D_F = 2\ a/2.$$

Here, $C_o$ is a constant.

As the correlation function is replaced with the log function as shown in Equation 4, the graph illustrating the value of correlation function $C(t-t_0)$ is changed to a log dimension as shown in FIG. 8.

FIG. 8 is a graph illustrating conversion of the correlation to the log dimension according to the exemplary embodiment of the present invention. In the graph of FIG. 8, the vertical axis denotes a log value of the correlation function $C(t-t_0)$ and the horizontal axis denotes a log value of $(t-t_0)$.

Thus, a slope of the graph shown in FIG. 8 corresponds to the variable a according to FIG. 4. In this case, the slope (i.e., the variable a) is determined by a start location $t_u$ and a variable time length t at the start location (S507).

That is, the fractal function $dD_F$ is a function of the start location $t_u$ and the variable time $\Delta t$, and therefore the fractal function dDp can be represented as $dD_F(t_u, t)$.

Therefore, the diagnosis unit 140 performs calculation of a fractal dimension value using the fractal function $dD_F(t_u, t)$.

In further detail, the diagnosis unit 140 first changes $t_u$ while fixing t to a predetermined value and calculates $dD_F(t_u, t)$ according to the variable $t_u$ so as to acquire value of the fractal function corresponding to an arbitrarily variable $t_u$ and t (S508).

The $dD_F(t_u, t)$ calculated through the step S508 is represented as a non-linear curved line as shown in FIG. 9. FIG. 9 is a graph for determining a start time through $D_F$-values while a variable time is fixed according to the exemplary embodiment of the present invention.

As shown in FIG. 9, the values of $dD_F(t_u, t)$ are non-linearly and irregularly changed when changing $t_u$ while $\Delta t$ is fixed.

In such a non-linear curved line, the diagnosis unit 140 determines a time $t_{u1}$ of a minimal value point of $dD_F(t_u, t)$ (S509).

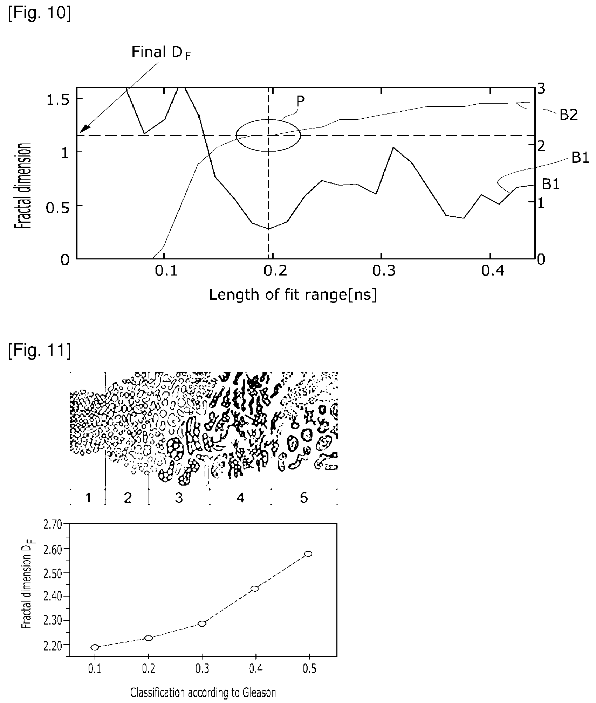

Next, the diagnosis unit 140 calculates values of $dD_F(t_u, t)$ by changing the variable time length t while fixing $t_u$ to $t_{u1}$ with respect to $dD_F(t_u, t)$ (S510), and the calculated $dD_F(t_{u1}, t)$ is represented as the non-linear curved line B1 in FIG. 10.

The diagnosis unit 140 determines a minimal value of the values of $dD_F(t_{u1}, t)$, represented as B1 in FIG. 10 as a $D_F$-value with respect to the measured value I(t).

In this case, the diagnosis unit 140 makes curved line B in FIG. 10 by differentiating $dD_F(t_{u1}, t)$ so as to acquire an accurate minimal value in the non-linear curved line B1 of FIG. 10 S511).

FIG. 10 is a graph for determining $D_F$-values through $D_F$-values and differentiation values while the start time is fixed according to the exemplary embodiment of the present invention.

In FIG. 10, the vertical axis denotes $D_F$-values and the horizontal axis denotes the variable time length $\Delta t$. In FIG. 10, the minimum value of the curved line B1 indicates a flat period in the curved line B2 by differentiation $dD_F(\Delta T)/d\Delta t$.

Thus, the diagnosis unit 140 finds a fractal dimension value having the minimum $(dD_F(\Delta T)/d\Delta t)$ in the flat period of the curved line B2 (S512), and determines the minimum value as a $D_F$-value of the sample A (S513).

In a second step the parameter C1 in Equation 3 was chosen by the condition, that the averaged value of $D_F$ averaging over a certain region of the parameter $t_u$ and t. Then the procedure is will be repeated as described above.

AS shown in FIG. 4, the diagnosis unit 140 calculates the fractal dimension value $D_F$ of the sample A and determines a Gleason grade corresponding to the fractal dimension values $D_F$ so that the diagositic device 140 can calculate a Gleason score and provide a diagnosis result on prediction of prostate cancer corresponding to the Gleason score.

The above-mentioned exemplary embodiments of the present invention are not embodied only by a method and apparatus. Alternatively, the above-mentioned exemplary embodiments may be embodied by a program performing functions that correspond to the configuration of the exemplary embodiments of the present invention, or a recording medium on which the program is recorded.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

<Description of symbols>

    100: in-vivo diagnosis device 110: light source unit
    120: optical unit 130: detection unit
    140: diagnosis unit

**Claims**

1. A prostate cancer diagnosis device using a fractal dimension value DF, comprising:

   a semiconductor laser (110) emitting a laser beam;
   an endoscope (150) transmitting the laser beam to a biopsy tissue in a human body and receiving auto-fluorescence generated from the biopsy tissue;
   an optical unit (120) transmitting the laser beam to the endoscope and receiving an auto-fluorescence generated from the biopsy tissue from the endoscope;
   a detection unit (130) detecting the auto-fluorescence received by the optical unit and measuring the intensity of the time-dependent autofluorescence in form of an auto-fluorescence measured value by a Time Correlation Single Photon method; and
   a diagnosis unit (140) calculating DF- values of the auto-fluorescence measured value using a fractal dimension algorithm,
   wherein the fractal dimension algorithm comprises: when the auto-fluorescence measured value is I(t), selecting a maximum I(t) that is higher than a predetermined reference intensity Iref as a target; approximation modeling the selected I(t) to a linear value using a modeling function F(t); calculating a correlation function that indicates a difference obtained by subtracting a measured value of an attenuation period of I(t) from the maximum value Fmax of F(t); modeling the correlation function with a fractal model and then replacing the modeled correlation function with a log function; calculating a fractal dimension function corresponding to a slope of a curve represented by values of the log function, the slope being determined by a start time and a variable time length; while the variable time length is fixed with respect to the fractal dimension function dDF(tu, t), calculating a first fractal dimension function value by changing the start time and determining a first start time corresponding to the minimal value of the calculated first fractal dimension function value; and while the start time is fixed to the first start time with respect to the fractal dimension function, calculating a second fractal dimension function value by changing the variable time length and determining the minimal value of the calculated second fractal dimension function value as the fractal dimension value.

2. The prostate cancer diagnosis device of claim 1, wherein the fractal dimension algorithm further comprises differentiating the second fractal dimension function value and determining the minimal value in a flat period of the differentiated second fractal dimension function as the fractal dimension value DF.

3. The prostate cancer diagnosis device of claim 2, wherein the diagnosis unit further includes a function for determining a Gleason grade corresponding to the calculated fractal dimension values and calculating a Gleason score using the determined Gleason grade.

4. The prostate cancer diagnosis device of claim 2, wherein the semiconductor laser emits a laser beam of a 375nm wavelength.

5. The prostate cancer diagnosis device of claim 3, wherein the semiconductor laser emits two laser beams of a 750nm wavelength with a time interval of picoseconds to femtoseconds.

6. The prostate cancer diagnosis device of claim 1, wherein the autofluorescence is fluorescence emitted from a unique fluorescence material, i.e., NADH, generated from cancer cells.

**Patentansprüche**

1. Prostatakrebs-Diagnosevorrichtung, die einen fraktalen Dimensionswert DF verwendet, enthaltend:

   einen Halbleiterlaser (110), der einen Laserstrahl emittiert;
   ein Endoskop (150), das den Laserstrahl zu einem Biopsie-Gewebe in einem menschlichen Körper aussendet und Autofluoreszenz empfängt, die von dem Biopsie-Gewebe generiert wird;
   eine optische Einheit (120), die den Laserstrahl zu dem Endoskop überträgt und eine Autofluoreszenz, die von dem Biopsie-Gewebe generiert wird, von dem Endoskop empfängt;
   eine Detektionseinheit (130), die die Autofluoreszenz, die von der optischen Einheit empfangen ist, detektiert und die Intensität der zeitabhängigen Autofluoreszenz in Form eines gemessenen Autofluoreszenzwerts durch ein Verfahren gemäß einer Zeitkorrelationseinzelphoton-Methode bzw. *"Time Correlation Single Photon"*-Methode misst; und
   eine Diagnoseeinheit (140), die DF-Werte des gemessenen Autofluoreszenzwerts unter Verwendung eines fraktalen Dimensions-Algorithmus misst;

   wobei der fraktale Dimensions-Algorithmus aufweist:

   wenn der gemessene Autofluoreszenzwert gleich I(t) ist, Wählen eines Maximums I(t), das höher ist als eine vorbestimmte Referenzintensität Iref, als ein Ziel; Approximationsmodellieren des ausgewählten I(t) auf einen linearen Wert unter Verwendung einer Modellierungsfunktion F(t); Berechnen einer Korrelationsfunktion, die eine Differenz anzeigt, die durch Subtrahieren eines gemessenen Werts einer Dämpfungsperiode von I(t) von dem maximalen Wert Fmax von F(t) erhalten ist; Modellieren der Korrelationsfunktion mittels eines fraktalen Modells und anschließendes Ersetzen der modellierten Korrelationsfunktion durch eine Log-Funktion; Berechnen einer fraktalen Dimensionsfunktion entsprechend einer Steigung einer Kurve, die durch Werte von der Log-Funktion repräsentiert ist, wobei die Steigung durch eine Startzeit und eine variable Zeitlänge bestimmt ist; Berechnen, während die variable Zeitlänge mit Bezug zu der fraktalen Dimensionsfunktion dDF(tu, t) fixiert ist, eines ersten Werts der fraktalen Dimensionsfunktion durch Ändern der Startzeit und Bestimmen einer ersten Startzeit, die dem minimalen Wert des berechneten ersten Werts der fraktalen Dimensionsfunktion entspricht; und Berechnen, während die Startzeit auf die erste Startzeit mit Bezug zu der fraktalen Dimensionsfunktion fixiert ist, eines zweiten Werts der fraktalen Dimensionsfunktion durch Ändern der variablen Zeitlänge und Bestimmen des minimalen Werts des berechneten zweiten Werts der fraktalen Dimensionsfunktion als den fraktalen Dimensionswert.

2. Prostatakrebs-Diagnosevorrichtung nach Anspruch 1, bei der der fraktale Dimensions-Algorithmus weiterhin das Differenzieren des zweiten Werts der fraktalen Dimensionsfunktion und das Bestimmen des minimalen Werts in einer flachen Periode der differenzierten zweiten fraktalen Dimensionsfunktion als den fraktalen Dimensionswert DF aufweist.

3. Prostatakrebs-Diagnosevorrichtung nach Anspruch 2, bei der die Diagnoseeinheit ferner eine Funktion für die Bestimmung eines Gleason-Grads entsprechend den berechneten fraktalen Dimensionswerten und für die Berechnung eines Gleason-Scores unter Verwendung des bestimmten Gleason-Grads aufweist.

4. Prostatakrebs-Diagnosevorrichtung nach Anspruch 2, bei der der Halbleiterlaser einen Laserstrahl mit einer Wellenlänge von 375 nm aussendet.

5. Prostatakrebs-Diagnosevorrichtung nach Anspruch 3, bei der der Halbleiterlaser zwei Laserstrahlen mit einer Wellenlänge von 750 nm mit einem Zeitintervall von Pikosekunden bis Femtosekunden aussendet.

6. Prostatakrebs-Diagnosevorrichtung nach Anspruch 1, bei der die Autofluoreszenz eine Fluoreszenz ist, die von einem einzigartigen Fluoreszenzmaterial, das heißt NADH, ausgesendet wird, das aus Krebszellen generiert ist.

## Revendications

1. Dispositif de diagnostic du cancer de la prostate utilisant une valeur de dimension fractale DF, comprenant :

   un laser à semi-conducteur (110) émettant un faisceau laser ;
   un endoscope (150) transmettant le faisceau laser à un tissu de biopsie dans un corps humain et recevant une auto-fluorescence générée à partir tissu de biopsie ;
   une unité optique (120) transmettant le faisceau laser à l'endoscope et recevant une auto-fluorescence générée à partir du tissu de biopsie en provenance de l'endoscope ;
   une unité de détection (130) détectant l'auto-fluorescence reçue par l'unité optique et mesurant l'intensité de l'auto-fluorescence dépendant du temps sous la forme d'une valeur mesurée d'auto-fluorescence par un procédé à photons individuels à corrélation temporelle ; et
   une unité de diagnostic (140) calculant des valeurs DF de la valeur mesurée d'auto-fluorescence à l'aide d'un algorithme de dimension fractale,
   dans lequel l'algorithme de dimension fractale comprend : quand la valeur mesurée d'auto-fluorescence est I(t), la sélection d'un maximum I(t) qui est supérieur à une intensité de référence prédéterminée Iref comme cible ; la modélisation par approximation du I(t) sélectionné en une valeur linéaire à l'aide d'une fonction de modélisation F(t) ; le calcul d'une fonction de corrélation qui indique une différence obtenue en soustrayant une valeur mesurée d'une période d'atténuation de I(t) de la valeur maximale Fmax de F(t) ; la modélisation de la fonction de corrélation avec un modèle fractal puis le remplacement de la fonction de corrélation modélisée par une fonction log ; le calcul d'une fonction de dimension fractale correspondant à une pente d'une courbe représentée par des valeurs de la fonction log, la pente étant déterminée par un temps de départ et par une longueur de temps variable ; alors que la longueur de temps variable est fixe par rapport à la fonction de dimension fractale dDF(tu, t), le calcul d'une première valeur de fonction de dimension fractale en changeant le temps de départ et la détermination d'un premier temps de départ correspondant à la valeur minimale de la première valeur de fonction de dimension fractale calculée ; et alors que le temps de départ est fixé au premier temps de départ par rapport à la fonction de dimension fractale, le calcul d'une deuxième valeur de fonction de dimension fractale en changeant la longueur de temps variable et la détermination de la valeur minimale de la deuxième valeur de fonction de dimension fractale calculée comme valeur de dimension fractale.

2. Dispositif de diagnostic du cancer de la prostate selon la revendication 1, dans lequel l'algorithme de dimension fractale comprend en outre la différenciation de la deuxième valeur de fonction de dimension fractale et la détermination de la valeur minimale dans une période plate de la deuxième fonction de dimension fractale différenciée comme valeur de dimension fractale DF.

3. Dispositif de diagnostic du cancer de la prostate selon la revendication 2, dans lequel l'unité de diagnostic comprend en outre une fonction pour déterminer un grade de Gleason correspondant aux valeurs de dimension fractale calculées et le calcul d'un score de Gleason à l'aide du grade de Gleason déterminé.

4. Dispositif de diagnostic du cancer de la prostate selon la revendication 2, dans lequel le laser à semi-conducteur émet un faisceau laser d'une longueur d'onde de 375 nm.

5. Dispositif de diagnostic du cancer de la prostate selon la revendication 3, dans lequel le laser à semi-conducteur émet deux faisceaux laser d'une longueur d'onde de 750 nm avec un intervalle de temps allant de picosecondes à des femtosecondes.

6. Dispositif de diagnostic du cancer de la prostate selon la revendication 1, dans lequel l'auto-fluorescence est une fluorescence émise à partir d'un matériau à fluorescence unique, c'est-à-dire du NADH, généré à partir de cellules cancéreuses.

[Fig. 1]

110       120       130       140   100

| Light source unit | → | Optical unit | | Detection unit | → | Diagnosis unit |

Endoscope ~150

Probe

Prostate in human body

[Fig. 2]

Light source unit (110)      Optical unit(120)

40       130

Third condenser → Detection unit

110       10

Laser source → Beam expansion unit → Beam splitter unit ~20

Second condenser ~30

Endoscope ~150

[Fig. 3]

[Fig. 4]

```
                    ( Start )
                        │
                        ▼
        ┌──────────────────────────────┐
        │   Irradiate laser beam        │ ── S410
        │ (wavelength: 375nm or 750nm)  │
        └──────────────────────────────┘
                        │
                        ▼
        ┌──────────────────────────────┐
        │ Collect auto-fluorescence     │ ── S420
        │ reflected from biopsy tissue  │
        │     through endoscope         │
        └──────────────────────────────┘
                        │
                        ▼
        ┌──────────────────────────────┐
        │ Measure the amount of auto-   │ ── S430
        │ fluorescence using photon     │
        │     counting method           │
        └──────────────────────────────┘
                        │
                        ▼
        ┌──────────────────────────────┐
        │ Calculate DF value from       │
        │ measured result using fractal │ ── S440
        │ dimension algorithm           │
        │ store calculated DF value     │
        └──────────────────────────────┘
                        │
                        ▼          S450
┌────────────────┐ No  ╱───────────────────────╲
│ Next point or  │◄────  Is every measurement    ◄──
│     sample     │      ╲      finished?       ╱
└────────────────┘       ╲───────────────────╱
        S460                     │ Yes
                                 ▼
        ┌──────────────────────────────┐
        │ Determine Gleason grade       │ ── S470
        │ corresponding to calculated   │
        │          DF value             │
        └──────────────────────────────┘
                        │
                        ▼
        ┌──────────────────────────────┐
        │ Calculate Gleason score using │ ── S480
        │      two Gleason grades       │
        └──────────────────────────────┘
                        │
                        ▼
        ┌──────────────────────────────┐
        │ Determine diagnosis result    │ ── S490
        │ corresponding to Gleason score│
        └──────────────────────────────┘
                        │
                        ▼
        ┌──────────────────────────────┐
        │    Output diagnosis result    │ ── S500
        └──────────────────────────────┘
                        │
                        ▼
                    (  End  )
```

[Fig. 5]

```
                            ( Start )
                               │
  Measure intensity I(t) of auto-fluorescence of prostate sample    ─ S501
                               │
        Set I(t) of which Imax is greater than Ith as a target      ─ S502
                               │
     Perform approximate modeling I(t) in dotted waveform to F(t)   ─ S503
                               │
              Determine Fmax that is the maximum value of           ─ S504
               F(t) (a point of Fmax is set to to)
                               │
         Calculate correlation function value in attenuation period ─ S505
                               │
        Convert correlation function value to log function value to ─ S506
          acquire fractal dimension variable "a" (DF = 2-a/2)
                               │
           Fractal dimension variable "a" corresponds to slope of   ─ S507
                 log function graph, dDF(tu, Δt)
                               │
            Calculate DF value according to variable start time tu  ─ S508
                  while the start time Δt is fixed
                               │
       Determine first start time tu1 having the minimal DF-Value   ─ S509
                               │
           Calculate DF value according to variable time period Δt  ─ S510
                 while the first start time tu1 is fixed
                               │
                       Differentiate DF-Value                       ─ S511
                               │
               Determine minimum value in flat period               ─ S512
                               │
               Determine the determined minimum value as            ─ S513
                    fractal dimension DF-Value
                               │
                             ( End )
```

[Fig. 6]

Amount of light emission(Intensity)

● : I(t)
— : F(t)

$t_0$          Time(t)

[Fig. 7]

[Fig. 8]

[Fig. 9]

[Fig. 10]

Final D$_F$

[Fig. 11]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 201013254 A1

- WO 2009059072 A2

**Non-patent literature cited in the description**

- **C.E. GERICH.** *Detection of Cancer Cells in Prostate Tissue with Time-Resolved Fluorescence Spectroscopy*

- Optical Interactions with Tissue and Cells XXII. *SPIE,* 10 February 2011, vol. 7897 (1), 98225-6705